# EUROPEAN PATENT APPLICATION

(11) **EP 2 221 740 A1**
(43) Date of publication of application: **25.08.2010**
(21) Application number: 10152910.5
(22) Date of filing: 08.02.2010
(51) Int. Cl.: G06F 19/00

(54) **Digital image storage system and human body data comparison method for medical aesthetic applications**

(30) Priority: 18.02.2009 TW 98105080
(71) Applicant: Chen, Chun-Leon, Las Vegas, NV 89146 (US)
(72) Inventor: Cha, Jennifer, Las Vegas, NV 89117 (US)
(74) Representative: Graf Glück Habersack Kritzenberger

(57) **Abstract**

A human body data comparison method includes steps of a) picking up human body digital 3D image data from a person during the person's optimal lifetime of said person for use as reference human body digital 3D image data, b) storing the reference human body digital 3D image data in a databank, c) fetching the reference human body digital 3D image data from the databank during a medical/aesthetic treatment of the person,; and d) comparing the fetched reference human body digital 3D image data and then performing the medical/aesthetic treatment subject to the comparison result. The invention also provides a digital image storage system for use with the human body data comparison method.

## Description

### BACKGROUND OF THE INVENTION

### 1. Field of the Invention:

The present invention relates to a digital storage system for medical and aesthetic applications. The present invention relates also to a human body data comparison method for medical and aesthetic applications.

### 2. Description of the Related Art:

Currently, there are some health care organizations provide storage banks for storing health care-related substances such as embryonic stem cell, sperm, human egg, cord blood and etc., for future use as necessary. These health care-related substances are obtained from human bodies for specific purposes. To certain medical or aesthetic operations, the aforesaid storage banks make no contributions.

Before making a surgical or aesthetic operation, the patient may be unable to precisely describe the best mode, such as skin color, body segment ratio, face length, or the like. In this case, a miscommunication between the patent and the surgeon may occur. When this problem occurs, the best mode may be unable to achieve.

Therefore, it is desirable to provide an efficient way that enables the surgeon to compare reference data before performing an operation, thereby achieving the best mode.

### SUMMARY OF THE INVENTION

The present invention has been accomplished under the circumstances in view. It is the main object of the present invention to provide a human body data comparison method, which avoids a miscommunication between the patent and the medical or aesthetic plastic surgeon when taking a medical or aesthetic treatment.

To achieve this and other objects, a human body data comparison method includes steps of a) picking up human body digital 3D image data from a person during the person's optimal lifetime of said person for use as reference human body digital 3D image data, b) storing the reference human body digital 3D image data in a databank, c) fetching the reference human body digital 3D image data from the databank during a medical/aesthetic treatment of the person,; and d) comparing the fetched reference human body digital 3D image data and then performing the medical/aesthetic treatment subject to the comparison result. The invention also provides a digital image storage system for use with the human body data comparison method.

A digital image storage system for use with the aforesaid human body data comparison method comprises at least one human body image pickup unit adapted to pick up human body digital 3D image data of a person during the person's optimal lifetime, a data storage device adapted for storing the human body digital 3D image data picked up by the at least one human body image pickup unit, and a display device controllable to display the human body digital 3D image data when performing a medical/aesthetic treatment.

### BRIEF DESCRIPTION OF THE DRAWINGS

FIG. 1 is a system block diagram of a digital image storage system for medical and aesthetic applications.
FIG. 2 is a flow chart of a human body data comparison method for medical and aesthetic applications according to the present invention.

### DETAILED DESCRIPTION OF THE PREFERRED EMBODIMENT

The invention provides a digital image storage system and a human body data comparison method for medical and aesthetic applications.

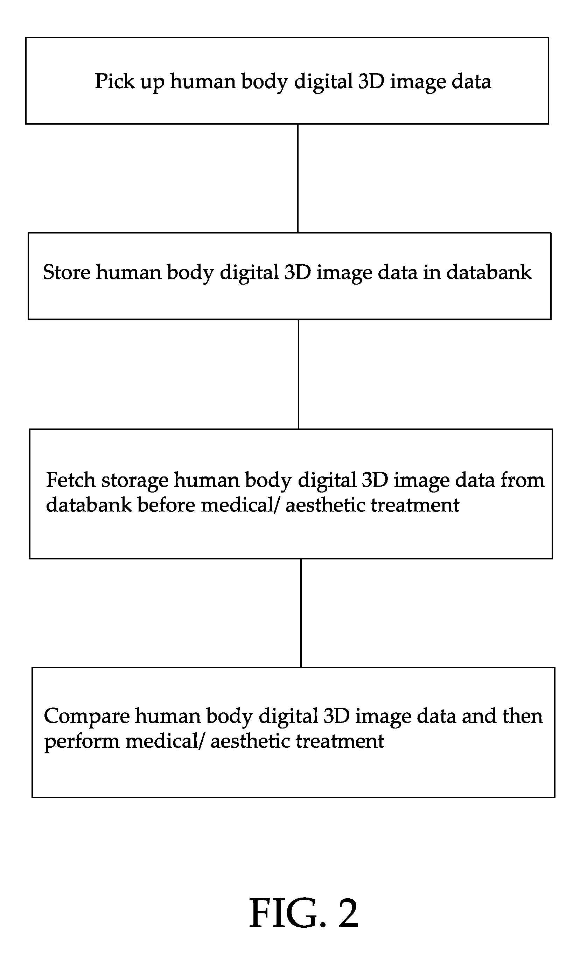

FIG. 2 is a flow chart of the human body data comparison method for medical and aesthetic applications according to the present invention. As illustrated, the human body data comparison method includes the steps of:
a) picking up human body digital 3D image data from a person during the person's optimal lifetime for use as reference human body digital 3D image data of the person;
b) storing the reference human body digital 3D image data in a databank;
c) fetching the reference human body digital 3D image data from the databank when the person is going to receive a medical/aesthetic treatment; and
d) comparing the fetched reference human body digital 3D image data and then performing the medical/aesthetic treatment subject to the comparison result.

In actual practice, the optimal lifetime of a person is during the age of 18-28. The reference human body digital 3D image data of a person is the image data of the person's skeleton data, tissue data and skin data obtained by means of scanning of a digital image pickup device. The reference human body digital 3D image data thus obtained in stored in a databank permanently, and classified subject to birthday, name, and personal ID. When the person is going to receive a medical or aesthetic treatment a certain period after the time in which the person's reference human body digital 3D image data was stored in the aforesaid databank, the surgeon can fetch the person's reference human body digital 3D image data from the databank for display and comparison and then perform a medical or aesthetic operation subject to the comparison result.

The teeth of a human body will wear with use. The skin color of a human body will change with age growing. The skeleton may shrink when a person is getting old. When one is going to receive a medical or aesthetic operation to resume one's best mode, the reference human body digital 3D image data can be fetched from the databank for reference by the surgeon, preventing a misunderstanding.

For example, when a person is going to whiten the skin, the aesthetic plastic surgeon can fetch the reference human body digital 3D image data from the databank to review the skin color of the person during his (her) optical lifetime.

For dental application, a dentist can fetch the early reference human body digital 3D image data of a person to check the original lower jaw height for reference during selection of an artificial tooth, assuring optical tooth transplantation.

Further, when performing a knee joint replacement to replace a painful damaged or diseased knee joint with an artificial joint, the surgeon can fetch the patient's early reference human body digital 3D image data from the databank for reference so as to select the best fit artificial joint for installation.

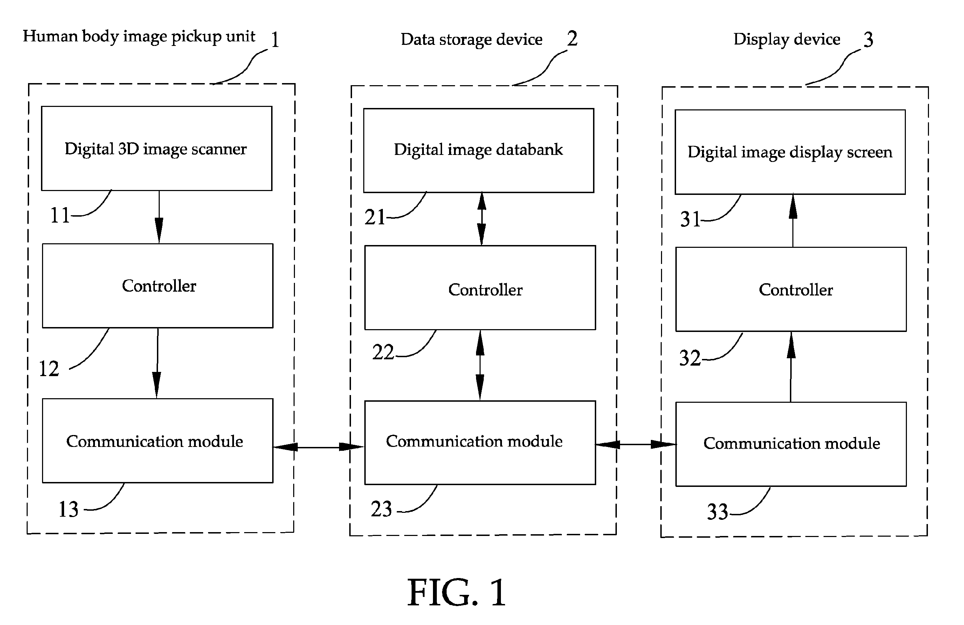

For use with the aforesaid human body data comparison method, the digital image storage system, as shown in FIG. 1, includes a human body image pickup unit **1,** a data storage device **2,** and a display device **3.** The human body image pickup unit **1** is adapted to obtain the human body digital 3D image data of a person during the person's optimal lifetime. The human body digital 3D image data thus obtained is then stored in the data storage device **2**. The human body digital 3D image data can be fetched from the data storage device **2** and then displayed on the display device **3** for reference by a surgeon when is going to person a medical or aesthetic treatment on the person.

Referring to FIG. 1 again, the human body image pickup unit **1** comprises at least one digital 3D image scanner **11** adapted to scan the human body of a person for obtaining digital 3D image data, a controller **12** adapted for converting the digital 3D image data thus obtained into a predetermined format, and a communication module **13** adapted for transmitting the formatted digital 3D image data from the controller **12** to the data storage device **2.** The digital 3D image data includes the image data of the skeleton, tissues and skin of the human body. The digital 3D image scanner **11** can be a CT (computer tomography), MRI (Magnetic resonance imaging) scanner, and digital camera.

The data storage device **2** comprises a digital image databank **21,** a controller **22,** and a communication module **23.** By means of the communication module **23,** the controller **22** receives the formatted digital 3D image data from the controller **12** of the human body image pickup unit **1** and stores the formatted digital 3D image data in the digital image databank **21.**

The display device **3** comprises a digital image display screen **31,** a controller **32,** and a communication module **33.** By means of communication between the communication module **33** of the display device **3** and the communication module **23** of the data storage device **2,** the controller **32** is controlled to fetch the formatted digital 3D image data from the digital image databank **21** of the data storage device **2** and to display the fetched digital 3D image data on the display screen **31.**

Although a particular embodiment of the invention has been described in detail for purposes of illustration, various modifications and enhancements may be made without departing from the spirit and scope of the invention. Accordingly, the invention is not to be limited except as by the appended claims.

## Claims

1. A digital image storage system for medical/aesthetic applications, comprising:
at least one human body image pickup unit (1) adapted to pick up human body digital 3D image data of a person during the person's optimal lifetime;
a data storage device (2) adapted for storing said human body digital 3D image data picked up by said at least one human body image pickup unit (1); and
a display device (3) controllable to display said human body digital 3D image data when performing a medical/aesthetic treatment.

2. The digital image storage system as claimed in claim 1, wherein each said human body image pickup unit (1) comprises a digital 3D image scanner (11) adapted to scan the human body of a person for obtaining digital 3D image data, a controller (12) adapted for converting the digital 3D image data thus obtained into a predetermined format, and a communication module (13) adapted for transmitting the formatted digital 3D image data from the controller (12) of the respective human body image pickup unit (1) to said data storage device (2).

3. The digital image storage system as claimed in claim 2, wherein said digital 3D image data comprises the image data of the skeleton, tissues and skin of the human body.

4. The digital image storage system as claimed in claim 2, wherein said data storage device (2) comprises a communication module (23) for communication with the communication module (23) of each said human body image pickup unit (1), a digital image databank (21) for storing said digital 3D image data, and a controller (22) for controlling the communication module (23) of said data storage device (2) to communication with the communication module (23) of one said human body image pickup unit (1) for enabling the formatted digital 3D image data to be stored in said digital image databank (21).

5. The digital image storage system as claimed in claim 4, wherein said display device (3) comprises a digital image display screen (31), a communication module (33) for communication with the communication module (33) of said data storage device (2), and a controller (32) controllable to fetch the formatted digital 3D image data from said digital image databank (21) of said data storage device (2) and to display the fetched digital 3D image data on said display screen.

6. A human body data comparison method, comprising the steps of:
a) picking up human body digital 3D image data from a person during an optimal lifetime of said person for use as reference human body digital 3D image data of said person;
b) storing said reference human body digital 3D image data in a databank;
c) fetching said reference human body digital 3D image data from said databank during a medical/aesthetic treatment of said person; and
d) comparing the fetched reference human body digital 3D image data and then performing the medical/aesthetic treatment subject to the comparison result.

7. The human body data comparison method as claimed in claim 6, wherein said human body digital 3D image data comprises the image data of the skeleton, tissues and skin of the human body.
